# EUROPEAN PATENT SPECIFICATION

(11) **EP 3 741 342 B1**
(45) Date of publication and mention of the grant of the patent: **26.11.2025**
(21) Application number: 18901626.4
(22) Date of filing: 03.12.2018
(51) Int. Cl.: A61F 13/56, A61F 13/49, A61F 13/493, A61F 13/494, A61F 13/514, A61F 13/62

(54) **DISPOSABLE DIAPER**
WEGWERFWINDEL
COUCHE JETABLE

(30) Priority: 16.01.2018 JP 2018004989
(43) Date of publication of application: 25.11.2020
(73) Proprietor: Daio Paper Corporation, Ehime 799-0492 (JP)
(72) Inventor: OSHIMA, Aya, Sakura-shi, Tochigi 329-1411 (JP)
(74) Representative: Meissner Bolte Partnerschaft mbB
(86) International application number: PCT/JP2018/044355
(87) International publication number: WO 2019/142515

(56) References cited:
- WO-A1-2013/089198
- JP-A- 2011 078 491
- JP-A- 2011 200 336
- JP-A- 2011 200 336
- JP-A- 2016 214 713
- JP-A- 2017 176 677
- JP-A- 2017 176 677
- JP-B2- 4 004 814
- US-A1- 2008 077 102

## Description

### Technical Field

The present invention relates to a disposable diaper, and particularly to a disposable diaper in which a fastening tape can be easily cross-fastened.

### Background Art

Conventionally, there are Patent Literatures 1 and 2 relating to a disposable diaper. Patent Literature 1 discloses a technique relating to a disposable diaper that can guide a user to fix first and second tape fasteners to a target tape in a state where the first and second tape fasteners are inclined. Patent Literature 1 describes that this technique makes it possible to prevent excrement from leaking from a leg-surrounding opening of the disposable diaper, and to prevent the entire disposable diaper from slipping down when the disposable diaper is worn. However, since the first and second tape fasteners are fixed to the target tape in a state where the first and second tape fasteners are inclined, floating wrinkles are formed on both side portions, and it is difficult to securely fit the disposable diaper to a wearer.

Patent Literature 2 discloses a technique relating to a disposable diaper in which a hook tape is hardly peeled off from a sheet substrate, hardly damages the skin of a wearer, and has improved usability. According to this technique, even if an ear portion repeatedly expands and contracts when the disposable diaper is worn, an engagement member disposed on the ear portion is hardly peeled off. Patent Literature 2 describes that since the engagement member is not peeled off, there is no risk that a corner of the engagement member is peeled off, comes into contact with the skin of a wearer, and damages the skin. However, when the fastening tape is to be cross-fastened, a part of the fastening tape in which an elastically stretchable member is disposed is easily curled, and it is difficult to securely fit the disposable diaper to a wearer.

Patent Literature 3 discloses a disposable diaper comprising a liquid pervious top sheet; a liquid impervious back sheet; an absorber disposed between the top sheet and the back sheet; and an outer sheet disposed from a dorsal side to a ventral side on a back surface of the back sheet, wherein a fastening tape extending in a direction orthogonal to a dorsoventral direction is disposed in a dorsal section of the outer sheet, the fastening tape includes:
a base portion fixed to the outer sheet; a main unit section located on a distal end of the base portion; and an engagement portion located on a distal end of the main unit section a dorsal end edge of the main unit section is continuous with a dorsal end edge of the engagement portion, a ventral end edge of the main unit section is continuous with a ventral end edge of the engagement portion, the fastening tape has a slit portion formed in an extending direction, the main unit section has elastically stretchable members, arranged in the extending direction at intervals in the dorsoventral direction, and a ventral portion and a dorsal portion in the engagement portion extend more distally than a central portion thereof.

Patent literature 4 discloses a very similar disposable diaper and is considered as closest prior art to the present invention.

### Citation List

### Patent Literature

Patent Literature 1: JP 2006-68281 A
Patent Literature 2: JP 2012-120663 A
Patent Literature 3: JP 2017-176677 A
Patent Literature 4: JP 2011-200336 A

### Summary of Invention

### Technical Problem

When it is difficult to securely fit a disposable diaper to a wearer, excrement may leak from a leg-surrounding opening, or the disposable diaper may slip down when the disposable diaper is worn.

Therefore, a main object to be achieved by the present invention is to provide a disposable diaper including a fastening tape that can reliably prevent floating wrinkles of a fastening tape and curling thereof. Another object is to provide a disposable diaper in which a fastening tape is unconsciously separated into an upper side and a lower side by a user unfamiliar with cross-fastening, and the user is encouraged to perform cross-fastening.

### Solution to Problem

The present invention solving the above problem is defined in claim 1.

### Brief Description of Drawings

Fig. 1 is an inner plan view obtained by developing a disposable diaper.
Fig. 2 is an external plan view obtained by developing a disposable diaper.
Fig. 3 is a cross-sectional view taken along line iii-iii of Fig. 1.
Fig. 4 is a cross-sectional view taken along line iv-iv of Fig. 1.
Fig. 5 is a cross-sectional view taken along line v-v of Fig. 1.
Fig. 6(a) is an inner plan view of a fastening tape of a first embodiment, and Fig. 6(b) is a cross-sectional view thereof taken along line A-A.
Fig. 7 is a detailed explanatory view of an engagement portion of the fastening tape of the first embodiment.
Fig. 8 is a conceptual diagram of the degree of stretch of an elastically stretchable member of the fastening tape of the first embodiment.
Fig. 9 is a plan view illustrating a procedure for separating and stretching a fastening tape.
Fig. 10 is a plan view of a fastening tape of a second embodiment.
Fig. 11 is a plan view of a fastening tape of a third embodiment.
Fig. 12 is a plan view of a fastening tape of a fourth embodiment.
Fig. 13 is a plan view illustrating stretch of a fastening tape, not forming part of the invention.
Fig. 14 is a plan view illustrating a cutting portion of a fastening tape.
Fig. 15 is a conceptual diagram when a disposable diaper is worn.

### Description of Embodiments

### <Disposable diaper>

A disposable diaper including a fastening tape capable of preventing floating wrinkles and curling and providing excellent cross-fastening will be described with reference to the attached drawings.

### (Meaning of terms, and the like)

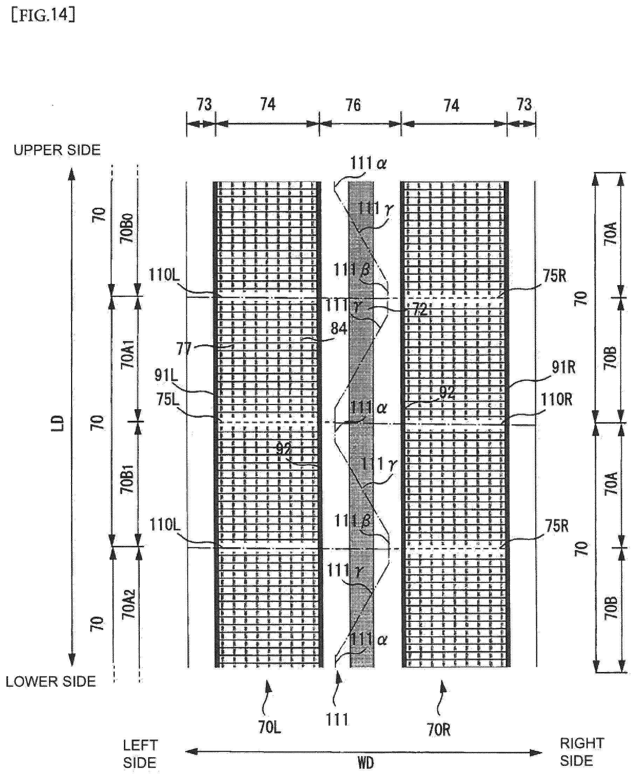

Here, "dorsoventral direction" refers to a direction connecting a ventral side to a dorsal side, "width direction" refers to a direction orthogonal to the dorsoventral direction, "up-down direction" refers to a direction orthogonal to a waist direction in a state where a disposable diaper is worn, "inner surface" refers to a body side surface of each member, and "outer surface" refers to a surface on the opposite side to the body side. "Upper side" refers to a dorsal side in a dorsoventral direction, and "lower side" refers to a ventral side in the dorsoventral direction. "Manufacturing direction LD" refers to a manufacturing direction of a fastening tape 70 particularly as illustrated in Fig. 14, and coincides with a direction connecting a ventral side to a dorsal side. "Manufacturing width direction WD" refers to a direction orthogonal to the manufacturing direction LD.

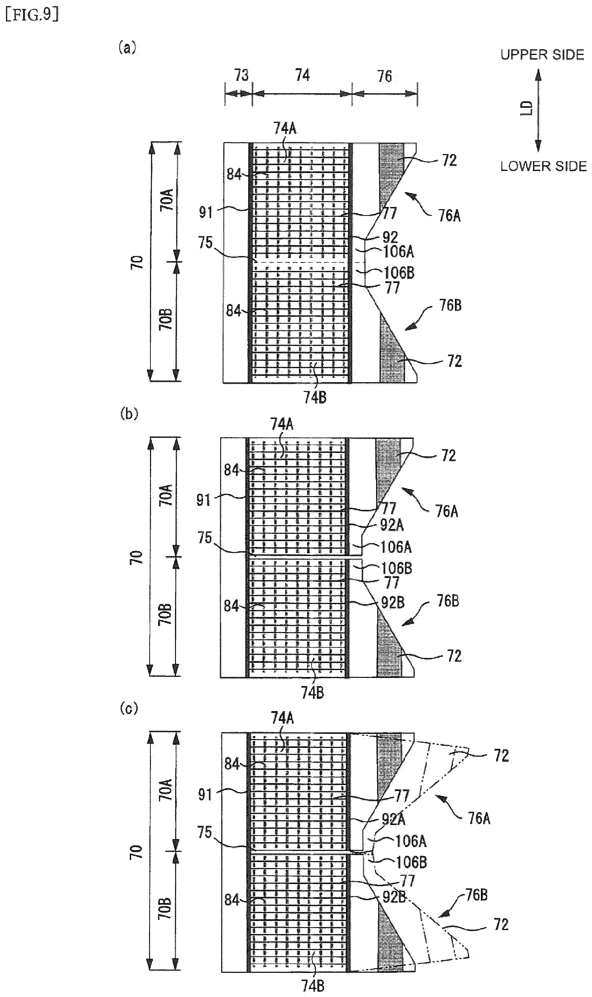

As illustrated in Fig. 9, an upper fastening tape 70A refers to a site of the fastening tape 70 located on a dorsal side of a slit portion 75 of a substrate sheet 71 after the fastening tape 70 is cut at the slit portion 75, and a lower fastening tape 70B refers to a site of the fastening tape 70 located on a ventral side of the slit portion 75 of the substrate sheet 71 after the fastening tape 70 is cut at the slit portion 75.

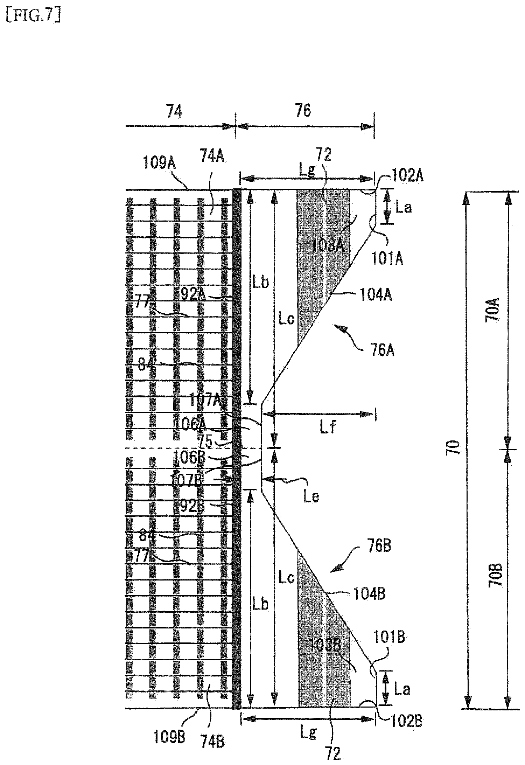

Regarding the name of each part of an engagement portion 76A, as illustrated in Fig. 7, an upper edged of the engagement portion is referred to as an engagement portion upper end edge 102A, an outer edge of the engagement portion is referred to as an engagement portion outer edge 101A, and an inclined portion from a grip portion 103A to a narrowing portion 106A is referred to as an engagement portion inclined edge 104A. Regarding the name of each part of an engagement portion 76B, a lower side of the engagement portion is referred to as an engagement portion lower end edge 102B, an outer side of the engagement portion is referred to as an engagement portion outer edge 101B, and an inclined portion from a grip portion 103B to a narrowing portion 106B is referred to as an engagement portion inclined edge 104B.

Upper sides of a bonded portion 92A and a bonded portion 92B refer to upper sides of the bonded portion 92A and the bonded portion 92B extending in the manufacturing direction LD in Figs. 9(b) and 9(c), respectively. Specifically, the upper side of the bonded portion 92A refers to an upper side of the fastening tape 70A, and the upper side of the bonded portion 92B refers to the vicinity of the slit portion 75. Lower sides of the bonded portion 92A and the bonded portion 92B refer to lower sides of the bonded portion 92A and the bonded portion 92B extending in the manufacturing direction LD in Figs. 9(b) and 9(c), respectively. Specifically, the lower side of the bonded portion 92A refers to the vicinity of the slit portion 75, and the lower side of the bonded portion 92B refers to a lower side of the fastening tape 70B.

Upper sides of a main unit section 74A and a main unit section 74B refer to upper sides of the main unit section 74A and the main unit section 74B extending in the manufacturing direction LD in Figs. 9(b) and 9(c), respectively. Specifically, the upper side of the main unit section 74A refers to an upper side of the fastening tape 70A, and the upper side of the main unit section 74B refers to the vicinity of the slit portion 75. Lower sides of the main unit section 74A and the main unit section 74B refer to lower sides of the main unit section 74A and the main unit section 74B extending in the manufacturing direction LD in Figs. 9(b) and 9(c), respectively. Specifically, the lower side of the main unit section 74A refers to the vicinity of the slit portion 75, and the lower side of the main unit section 74B refers to a lower side of the fastening tape 70B.

### (Formation of disposable diaper 100)

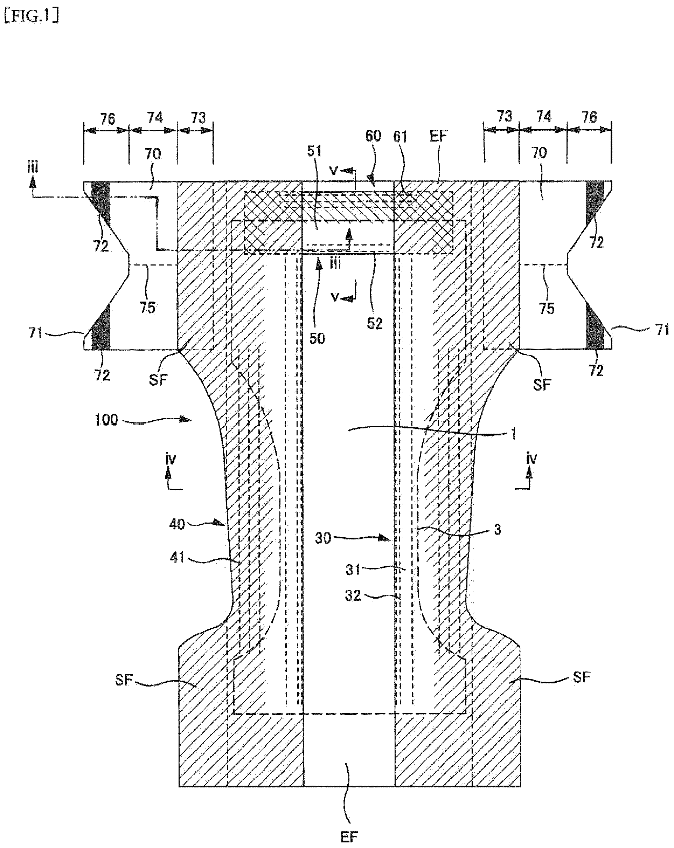

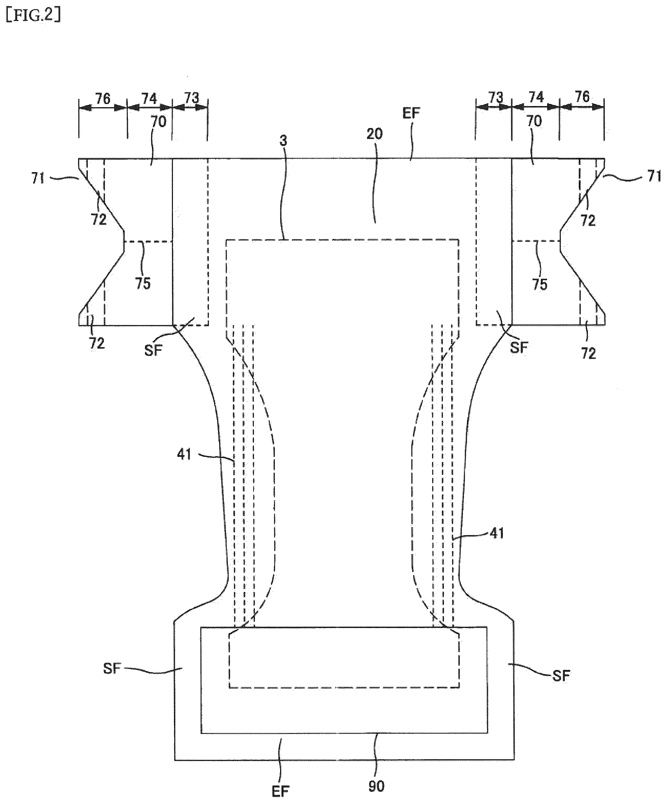

As illustrated in Figs. 1 and 2, a disposable diaper 100 includes a liquid pervious top sheet 1 on a body side, a liquid impervious back sheet 2 on the side opposite to the body side, and an absorber 3 disposed between the top sheet 1 and the back sheet 2. An outer sheet 20 is disposed on an outer surface of the back sheet 2.

On outer sides of the absorber 3 in the width direction, leg-surrounding three-dimensional gathers 30 are disposed in order to prevent excrement from leaking to the outside. On an outer side of a base portion of each of the three-dimensional gathers 30, leg-surrounding flat gathers 40 for preventing excrement from leaking to the outside are arranged at predetermined intervals.

On a dorsal side of the absorber 3 in the dorsoventral direction, a dorsal side three-dimensional gather 50 for preventing excrement from leaking to the outside is disposed. In a base portion of the three-dimensional gather 50, a dorsal side flat gather 60 for preventing excrement from leaking to the outside is disposed.

On outer sides of the absorber 3 in the dorsoventral direction, end flap portions EF to which the absorber 3 does not extend are disposed. On outer sides of the absorber 3 in the width direction, side flap portions SF to which the absorber 3 does not extend are disposed.

On dorsal sides of the side flap portions SF, the fastening tapes 70 extending toward the outside of the absorber 3 are disposed. On a ventral side of an outer surface of the outer sheet 20, a target sheet 90 to be engaged with the fastening tapes 70 when the disposable diaper 100 is worn by a wearer is disposed.

### (Top sheet 1)

In the present embodiment, the top sheet 1 extends to the outside of an outer peripheral edge of the absorber 3, and the extended site is fixed to an inner surface of the back sheet 2 via an adhesive such as a hot melt adhesive. Note that the form can be changed into a form in which the top sheet 1 does not extend to the outside of the outer peripheral edge of the absorber 3.

As the top sheet 1, a perforated or imperforated nonwoven fabric, a perforated plastic sheet, or the like is used. Examples of a material fiber constituting the nonwoven fabric include a synthetic fiber such as a polyolefin-based fiber including polyethylene and polypropylene, a polyester-based fiber, or a polyamide-based fiber, a regenerated fiber such as rayon or cupra, and a natural fiber such as cotton. Examples of a method for processing the nonwoven fabric include a known method such as a spunlace method, a spunbond method, an SMS method, a thermal bond method, a melt blown method, a needle punch method, an air through method, or a point bond method. The nonwoven fabric used for the top sheet 1 preferably has a fiber basis weight of 15 to 30 g/m², and a thickness of 0.05 to 1 mm.

### (Back sheet 2)

The back sheet 2 extends to the outside of the outer peripheral edge of the absorber 3 and blocks movement of excrement absorbed by the absorber 3 to the outside.

As the back sheet 2, in addition to a plastic film such as a polyethylene film, a sheet having moisture permeability without impairing a water blocking property can also be used from a viewpoint of preventing stuffiness. As the water blocking and moisture permeable sheet, a microporous sheet obtained by melt-kneading an inorganic filler in a polyolefin-based resin such as polyethylene or polypropylene to form a sheet, and then stretching the sheet in a monoaxial or biaxial direction can be used. The back sheet 2 preferably has a weight per unit area of 13 to 40 g/m², and a thickness of 0.01 to 0.1 mm.

### (Absorber 3)

As the absorber 3, it is possible to use basically a pulp fiber stack, an assembly of filaments such as cellulose acetate, or a nonwoven fabric, and as necessary, a material obtained by mixing or fixing super absorbent polymer therewith. The absorber 3 preferably has a pulp basis weight of 100 to 500 g/m², and a thickness of 1 to 15 mm. A superabsorbent polymer preferably has a basis weight of 0 to 300 g/m². When the content of the highly water-absorbing resin is too small, sufficient absorbability cannot be imparted. When the content of the superabsorbent polymer is too large, there is no entanglement between pulp fibers, and twisting, cracking, and the like occur easily.

The absorber 3 is formed in a single-layered structure, but can be formed in a two-layered structure including an upper-layer absorber on a body side and a lower-layer absorber on the side opposite to the body. The absorber 3 is formed in an hourglass shape in a plan view, but can be formed in a rectangular shape or the like. In order to prevent the superabsorbent polymer from falling off, the absorber 3 can also be wrapped with liquid pervious crepe paper.

### (Outer sheet 20)

The outer sheet 20 covers the back sheet 2 to impart an appearance and a texture like cloth to an outer surface of the disposable diaper 100. The outer sheet 20 is preferably formed of a nonwoven fabric. Examples of a material fiber thereof include a synthetic fiber such as a polyolefin-based fiber including polyethylene and polypropylene, a polyester-based fiber, or a polyamide-based fiber, a regenerated fiber such as rayon or cupra, and a natural fiber such as cotton. The outer sheet 20 can be manufactured by a processing method such as a spunlace method, a spunbond method, a thermal bond method, an air through method, or a needle punch method. However, a long-fiber nonwoven fabric such as a spunbonded nonwoven fabric, an SMS nonwoven fabric, or an SMMS nonwoven fabric is preferable from a viewpoint of achieving both texture and strength.

A single sheet of nonwoven fabric can be used, or a plurality of sheets of nonwoven fabric can also be used in a stack. When a plurality of sheets of nonwoven fabric is used in a stack, the sheets of nonwoven fabric are preferably fixed to each other via an adhesive such as a hot melt adhesive. When a nonwoven fabric is used, the nonwoven fabric has a fiber basis weight of preferably 10 to 50 g/m², particularly preferably 15 to 30 g/m².

### (Leg-surrounding three-dimensional gather 30)

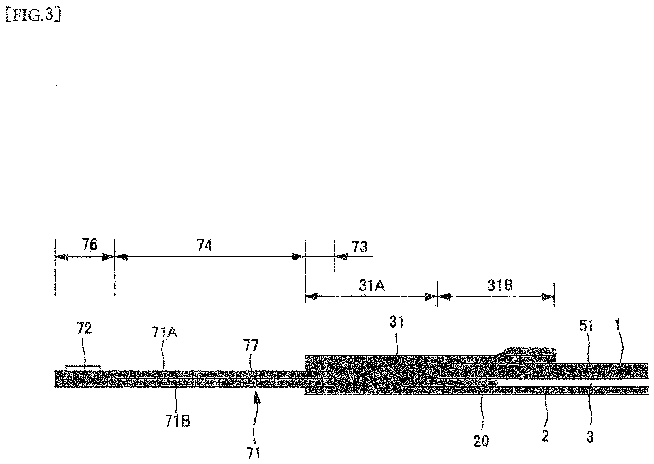

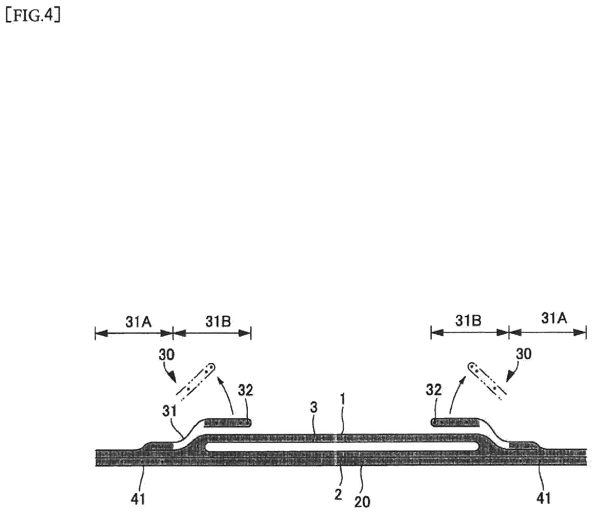

As illustrated in Figs. 3 and 4, a base portion 31A of a gather sheet 31 forming the leg-surrounding three-dimensional gather 30 is fixed to an outer portion of an inner surface of the top sheet 1 in the width direction and an outer portion of an inner surface of the back sheet 2 in the width direction in the dorsoventral direction. Both end portions of a standing part 31B of the gather sheet 31 in the dorsoventral direction are fixed to an outer portion of an inner surface of the top sheet 1 in the width direction and an outer portion of an inner surface of the gather sheet 51 forming the three-dimensional gather 50 in the width direction. An intermediate portion of the standing part 31B of the gather sheet 31 in the dorsoventral direction is not fixed to the inner surface of the top sheet 1 or the inner surface of the gather sheet 51 and is separated therefrom. In the standing part 31B, a plurality of elongated elastically stretchable members 32 extending in the dorsoventral direction is arranged at predetermined intervals in the width direction in a predetermined stretched state. As a result, when the disposable diaper 100 is worn by a wearer, the standing part 31B can stand toward a crotch portion of the wearer by a contraction force of the elastically stretchable members 32, and a distal portion of the standing part 31B can be pressed against the crotch portion of the wearer to prevent excrement from leaking to the outside.

As the gather sheet 31, in addition to a nonwoven fabric such as a spunbonded nonwoven fabric, a plastic film similar to that used for the back sheet 2, or a laminated sheet thereof can be used. However, a nonwoven fabric that has been subjected to a water-repellent treatment is preferable in view of touch to the skin.

As the elastically stretchable member 32, a usually used material such as a thread-like, string-like, or belt-like natural rubber or a synthetic rubber, for example, specifically, a polystyrene-based rubber, a polyolefin-based rubber, a polyurethane-based rubber, a polyester-based rubber, polyurethane, polyethylene, polystyrene, a styrene-butadiene copolymer, silicone, or polyester can be used. The elastically stretchable member 32 has a fineness of preferably about 500 to 1500 dtex, particularly preferably about 800 to 1300 dtex (preferably about 0.1 to 3 mm, particularly preferably about 0.5 to 3 mm in a case of natural rubber), and has a stretch ratio at the time of attachment of preferably 150 to 250%, particularly preferably about 160 to 200%. Note that in Fig. 1, the oblique lines diagonally rising to the right indicate fixing points of the gather sheet 31, the oblique lines diagonally rising to the left indicate fixing points of the gather sheet 51, and the dots in Figs. 3 to 5 indicate fixing sites with a hot melt adhesive or the like.

### (Leg-surrounding flat gather 40)

The flat gather 40 is disposed at a site of the side flap portion SF facing the base portion 31A of the gather sheet 31. In an outer portion of the back sheet 2 and the outer sheet 12 forming the side flap portion SF in the width direction, elongated elastically stretchable members 41 extending in the dorsoventral direction are arranged at predetermined intervals in the width direction in a predetermined stretched state. As a result, when the disposable diaper 100 is worn by a wearer, the flat gather 40 can be pressed against a leg portion of the wearer by a contraction force of the elastically stretchable members 41 to prevent excrement from leaking to the outside.

As the elastically stretchable member 41, a usually used material such as a thread-like, string-like, or belt-like natural rubber or a synthetic rubber, for example, specifically, a polystyrene-based rubber, a polyolefin-based rubber, a polyurethane-based rubber, a polyester-based rubber, polyurethane, polyethylene, polystyrene, a styrene-butadiene copolymer, silicone, or polyester can be used. The interval between the elastically stretchable members 41 is preferably about 2 to 15 mm, and particularly preferably about 3 to 7 mm. Furthermore, the elastically stretchable member 41 has a fineness of preferably about 500 to 1500 dtex, particularly preferably about 800 to 1300 dtex (preferably about 0.1 to 3 mm, particularly preferably about 0.5 to 3 mm in a case of natural rubber), and has a stretch ratio at the time of attachment of preferably 150 to 250%, particularly preferably about 160 to 200%.

### (Dorsal side three-dimensional gather 50)

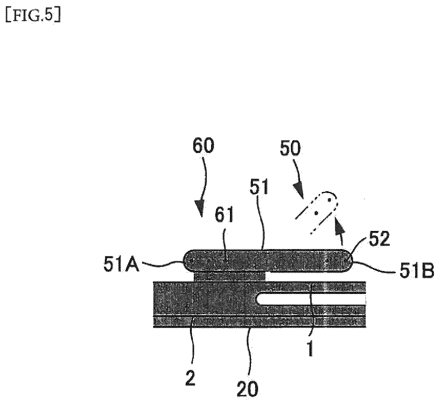

As illustrated in Fig. 5, a base portion 51A of the gather sheet 51 forming the dorsal side three-dimensional gather 50 is fixed to an intermediate portion of a dorsal section of an inner surface of the top sheet 1 in the width direction. Both side portions of an inner surface of a standing part 51B of the gather sheet 51 in the width direction are fixed to an outer surface of the gather sheet 31 facing an inner surface of the top sheet 1. In an intermediate portion of the standing part 51B of the gather sheet 51 in the width direction, a plurality of elongated elastically stretchable members 52 extending in the width direction is arranged at predetermined intervals in the dorsoventral direction in a predetermined stretched state. As a result, when the disposable diaper 100 is worn by a wearer, the standing part 51B can stand toward the back of the wearer by a contraction force of the elastically stretchable members 52, and a distal portion of the standing part 51B can be pressed against the back of the wearer to prevent excrement from leaking to the outside.

As the gather sheet 51, like the gather sheet 31, in addition to a nonwoven fabric such as a spunbonded nonwoven fabric, a plastic film similar to that used for the back sheet 2, or a laminated sheet thereof can be used. However, a nonwoven fabric that has been subjected to a water-repellent treatment is preferable in view of touch to the skin.

As the elastically stretchable member 52, like the elastically stretchable member 32, a usually used material such as a thread-like, string-like, or belt-like natural rubber or a synthetic rubber, for example, specifically, a polystyrene-based rubber, a polyolefin-based rubber, a polyurethane-based rubber, a polyester-based rubber, polyurethane, polyethylene, polystyrene, a styrene-butadiene copolymer, silicone, or polyester can be used. The elastically stretchable member 32 has a fineness of preferably about 500 to 1500 dtex, particularly preferably about 800 to 1300 dtex (preferably about 0.1 to 3 mm, particularly preferably about 0.5 to 3 mm in a case of natural rubber), and has a stretch ratio at the time of attachment of preferably 150 to 250%, particularly preferably about 160 to 200%.

### (Dorsal side flat gather 60)

The flat gather 60 is disposed at a site of the end flap portion EF facing the base portion 51A of the gather sheet 51. In the base portion 51A of the gather sheet 51, elongated elastically stretchable members 61 extending in the width direction are arranged at predetermined intervals in the dorsoventral direction in a predetermined stretched state. As a result, when the disposable diaper 100 is worn by a wearer, the flat gather 60 can be pressed against the back of the wearer by a contraction force of the elastically stretchable members 61 to prevent excrement from leaking to the outside.

As the elastically stretchable member 61, like the elastically stretchable member 41, a usually used material such as a thread-like, string-like, or belt-like natural rubber or a synthetic rubber, for example, specifically, a polystyrene-based rubber, a polyolefin-based rubber, a polyurethane-based rubber, a polyester-based rubber, polyurethane, polyethylene, polystyrene, a styrene-butadiene copolymer, silicone, or polyester can be used. The interval between the elastically stretchable members 41 is preferably about 2 to 15 mm, and particularly preferably about 3 to 7 mm. Furthermore, the elastically stretchable member 41 has a fineness of preferably about 500 to 1500 dtex, particularly preferably about 800 to 1300 dtex (preferably about 0.1 to 3 mm, particularly preferably about 0.5 to 3 mm in a case of natural rubber), and has a stretch ratio at the time of attachment of preferably 150 to 250%, particularly preferably about 160 to 200%. The elastically stretchable members 61 are disposed in the base portion 51A of the gather sheet 51. However, by disposing another sheet on a dorsal side of the gather sheet 51 in the dorsoventral direction, the elastically stretchable members 61 can be arranged therein.

### (Fastening tape 70)

As illustrated in Figs. 1 to 3, on dorsal sides of the side flap portions SF, the fastening tapes 70 extending to the outside are disposed. Each of the fastening tapes 70 includes a substrate sheet 71 and an engagement member 72 disposed on an inner surface of the substrate sheet 71.

### (Substrate sheet 71 of fastening tape)

A base portion 73 of the substrate sheet 71 is fixed between the outer sheet 20 and an outer portion of the gather sheet 31 in the width direction. In an intermediate portion of the main unit section 74 of the substrate sheet 71 in the dorsoventral direction, the slit portion 75 formed of perforations and the like is disposed. On an inner surface of the engagement portion 76 of the substrate sheet 71, the engagement member 72 is disposed.

The substrate sheet 71 is formed of an inner substrate sheet 71A located on the inside, an outer substrate sheet 71B located on the outside, and a plurality of elongated elastically stretchable members 77 arranged at predetermined intervals in the dorsoventral direction between the inner substrate sheet 71A and the outer substrate sheet 71B and being in a stretched state in the width direction. As a result, when the disposable diaper 100 is worn by a wearer, the disposable diaper 100 can be worn by the wearer while being in close contact with the wearer by a contraction force of the elastically stretchable members 77.

A material of each of the inner substrate sheet 71A and the outer substrate sheet 71B is preferably a nonwoven fabric, and any known nonwoven fabric can be used without any particular limitation. Examples of a fiber constituting the nonwoven fabric include a synthetic fiber such as a polyolefin-based fiber including polyethylene and polypropylene, a polyester-based fiber, or a polyamide-based fiber, a regenerated fiber such as rayon or cupra, and a natural fiber such as cotton. Examples of a method for manufacturing the nonwoven fabric include a known method such as a spunlace method, a spunbond method, an SMS method, a thermal bond method, a melt blown method, a needle punch method, an air through method, or a point bond method. In particular, a spunbonded nonwoven fabric and an SMS nonwoven fabric using a polyolefin-based fiber are preferable. The basis weight of a nonwoven fabric to be used can be determined as appropriate. However, the total basis weight of the nonwoven fabric in the main unit section is preferably 20 to 75 g/m², and particularly preferably 26 to 46 g/m². The total basis weight of the nonwoven fabric in each of a fixing portion and a distal portion is preferably 35 to 130 g/m², and particularly preferably 46 to 116 g/m². Within this range, the strength and rigidity of the base portion 73 fixed between the outer sheet 20 and the gather sheet 31 can be ensured, and the flexibility of the main unit section 74 and elasticity thereof can be ensured.

As the engagement member 72, a hook material of a mechanical fastener is preferable. The hook member has many engaging projections. Examples of the shapes of the engaging projections include (A) tick shape, (B) J shape, (C) mushroom shape, (D) T shape, and (E) double J shape (a shape in which the J-shaped ones are connected to each other back to back), and any one of these shapes may be used. An adhesive layer can also be used instead of the hook material.

The upper engagement portion 76A is disposed in an outer dorsal end portion of the substrate sheet 71 in the width direction, and the lower engagement portion 76B is disposed in an outer ventral end portion of the substrate sheet 71 in the width direction. As a result, as illustrated in Fig. 9(c), when the upper engagement portion 76A is stretched outward in the width direction, the upper fastening tape 70A can be further extended toward the ventral side as it is closer to the outside in the width direction. When the lower engagement portion 76B is stretched outward in the width direction, the lower fastening tape 70B can be further extended toward the dorsal side as it is closer to the outside in the width direction.

The length of the engagement portion 76 in the width direction is preferably formed so as to be 20 to 40% of the length of the main unit section 74. This can increase the degree of freedom of each of directions in which the upper fastening tape 70A and the lower fastening tape 70B are stretched, and the degree of freedom of a position where each of the engagement member 72 of the upper fastening tape 70A and the engagement member 72 of the lower fastening tape 70B is engaged with the target sheet 90.

As the elastically stretchable member 77, a usually used material such as a thread-like, string-like, or belt-like natural rubber or a synthetic rubber, for example, specifically, a polystyrene-based rubber, a polyolefin-based rubber, a polyurethane-based rubber, a polyester-based rubber, polyurethane, polyethylene, polystyrene, a styrene-butadiene copolymer, silicone, or polyester can be used. The elastically stretchable member 77 has a fineness of preferably about 300 to 3000 dtex, particularly preferably about 400 to 1300 dtex, more preferably about 900 to 1300 dtex, and has a stretch ratio at the time of attachment of preferably about 150 to 500%, particularly preferably about 200 to 400%, more preferably about 300 to 400%.

### (Target sheet 90)

The target sheet 90 is preferably a plastic film or a nonwoven fabric having many loop yarns on a surface thereof. This makes it possible to efficiently engage the engagement member 72 of the fastening tape 70 with the target sheet 90 when the disposable diaper 100 is worn by a wearer.

### <Fastening tape 70 of first embodiment>

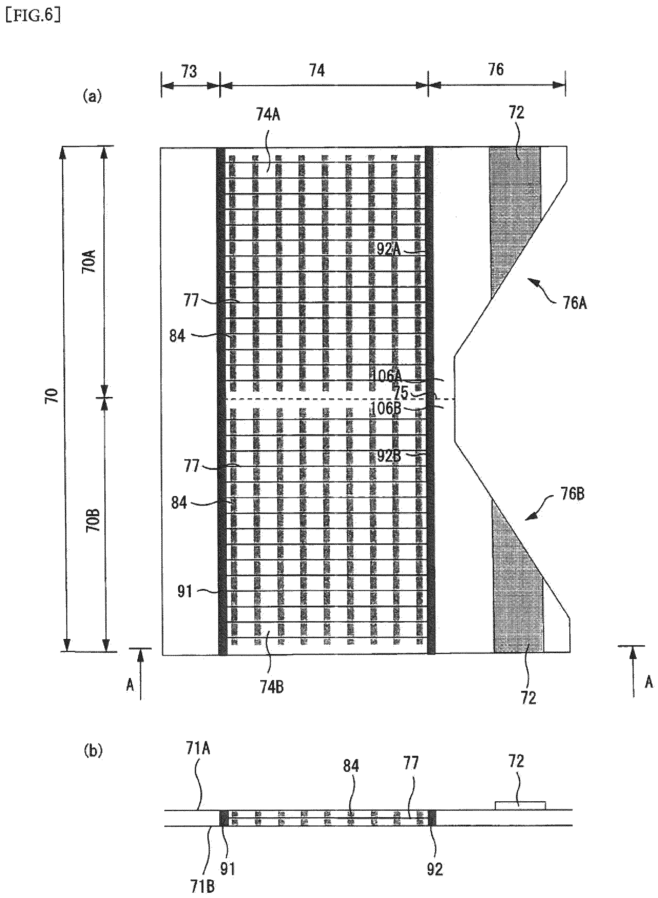

Next, a fastening tape 70 of a first embodiment illustrated in Fig. 6 will be described. Fig. 6(a) is an inner plan view, and Fig. 6(b) is a cross-sectional view taken along line A-A. A plurality of dots 84 arranged in the main unit section 74 in the dorsoventral direction in Fig. 6 are fixing points of the elastically stretchable members 77.

The fastening tape 70 of the first embodiment includes the base portion 73, the main unit section 74, and the engagement portion 76 from the inside to the outside in the width direction.

### (Main unit section 74)

The inner substrate sheet 71A and the outer substrate sheet 71B in the main unit section 74 each have many pleats so as to expand and contract in the width direction. Between the inner substrate sheet 71A and the outer substrate sheet 71B, the elastically stretchable members 77 are arranged at predetermined intervals in the width direction.

### (Engagement portion 76)

The engagement portion 76 will be described with reference to Fig. 7. The engagement portion 76 has a shape obtained by cutting out a trapezoid from a rectangle having the dorsoventral direction of the absorber 3 as a long side. Here, in the trapezoid, the top side is on the central side of the absorber 3 in a lateral direction, and the bottom side is on the outer side of the absorber 3 in the lateral direction. At this time, the lower bottom of the trapezoid is located on an imaginary line formed by connecting outermost edges of the engagement portion 76 in the dorsoventral direction, that is, engagement portion outer edges 101A and 101B. In other words, the engagement portion 76 can be said to have a shape formed by cutting an hourglass in half along a longitudinal center axis of the hourglass, that is, a semi-hourglass shape. The engagement portion 76A of the upper fastening tape 70A and the engagement portion 76B of the lower fastening tape 70B can be substantially line-symmetrical with an extension of the slit portion 75 as an axis.

### (Position of engagement portion 76A)

The engagement portion upper end edge 102A extends outward in the width direction from the upper end edge 109A of the main unit section 74A. That is, the engagement portion upper end edge 102A is located on an extension of the upper end edge 109A of the main unit section 74A. Therefore, the upper end edge of the upper fastening tape 70A extends linearly in a lateral direction from the main unit section 74A to the engagement portion 76A.

The length La of the engagement portion outer edge 101A is preferably shorter than at least a half of the length Lc of the upper fastening tape 70A in the dorsoventral direction. As a result, the grip portion 103A is disposed above and outside the upper fastening tape 70A. Therefore, a user grasps the grip portion 103A. Since the user grasps the grip portion 103A and stretches the upper fastening tape 70A outward in the width direction, the upper fastening tape 70A is curved and naturally goes down. Even a user unfamiliar with cross-fastening is encouraged to perform cross-fastening by the form of the engagement portion 76A.

The length Lb from the engagement portion upper end edge 102A to the narrowing portion of the semi-hourglass shape is longer than the length La of the engagement portion outer edge 101A.

The length Lb from the engagement portion upper end edge 102A to the narrowing portion of the semi-hourglass shape is preferably longer than a half of the length Lc of the upper fastening tape 70A in the dorsoventral direction. As a result, when a user grasps the grip portion 103A and stretches the upper fastening tape 70A outward in the width direction, substantially the entire bonded portion 92A extending in the dorsoventral direction receives an action of an extending force. If the length Lb from the engagement portion upper end edge 102A to the narrowing portion of the semi-hourglass shape is relatively short in relation to the length Lc of the upper fastening tape 70A in the dorsoventral direction, the action of the force received by the bonded portion 92A is deviated to an upper side of the bonded portion 92A. In this case, when a user grasps the grip portion 103A and stretches the upper fastening tape 70A outward in the width direction, floating wrinkles are formed in the width direction above the upper fastening tape 70A.

Note that the thickness Le of the narrowing portion of the semi-hourglass shape is shorter than the length Lg of the engagement portion upper end edge 102A. The details of the action of the force will be described later.

Furthermore, the length Lf from the engagement portion outer edge 101A to the narrowing portion of the semi-hourglass shape is preferably equal to or longer than a half of the length Lc of the upper fastening tape 70A in the dorsoventral direction. When the length Lf from the engagement portion outer edge 101A to the narrowing portion of the semi-hourglass shape is set in this manner, the area of the grip portion 103A becomes relatively large. A user can easily grasp the grip portion 103A and can easily stretch the upper fastening tape 70A outward in the width direction.

### (Position of engagement portion 76B)

The engagement portion lower end edge 102B extends outward in the width direction from the lower end edge 109B of the main unit section 74B. That is, the engagement portion lower end edge 102B is located on an extension of the lower end edge 109B of the main unit section 74B. Therefore, the lower end edge of the lower fastening tape 70B extends linearly in a lateral direction from the main unit section 74B to the engagement portion 76B.

The length La of the engagement portion outer edge 101B is preferably shorter than a half of the length Lc of the lower fastening tape 70B in the dorsoventral direction. As a result, the grip portion 103B is disposed below and outside the lower fastening tape 70B. Therefore, a user grasps the grip portion 103B. Since the user grasps the grip portion 103B and stretches the lower fastening tape 70B outward in the width direction, the lower fastening tape 70B is curved and naturally goes up. Even a user unfamiliar with cross-fastening is encouraged to perform cross-fastening by the form of the engagement portion 76B.

The length Lb from the engagement portion lower end edge 102B to the narrowing portion of the semi-hourglass shape is longer than the length La of the engagement portion outer edge 101B. The length Lb from the engagement portion lower end edge 102B to the narrowing portion of the semi-hourglass shape is preferably longer than a half of the length Lc of the lower fastening tape 70B in the dorsoventral direction. As a result, when a user grasps the grip portion 103B and stretches the lower fastening tape 70B outward in the width direction, substantially the entire bonded portion 92B extending in the dorsoventral direction receives an action of an extending force. If the length Lb from the engagement portion lower end edge 102B to the narrowing portion of the semi-hourglass shape is relatively shorter than the length Lc of the lower fastening tape 70B in the dorsoventral direction, the action of the force received by the bonded portion 92B is deviated to a lower side of the bonded portion 92B. In this case, when the user grasps the grip portion 103B and stretches the lower fastening tape 70B outward in the width direction, floating wrinkles are formed in the width direction above the lower fastening tape 70B.

Note that the thickness Le of the narrowing portion of the semi-hourglass shape is short in relation to the length Lg of the engagement portion lower end edge 102B. The details of the action of the force will be described later.

Furthermore, the length Lf from the engagement portion outer edge 101B to the narrowing portion of the semi-hourglass shape is preferably equal to or longer than a half of the length Lc of the lower fastening tape 70B in the dorsoventral direction. When the length Lf from the engagement portion outer edge 101B to the narrowing portion of the semi-hourglass shape is set in this manner, the area of the grip portion 103B becomes relatively large. A user can easily grasp the grip portion 103B and can easily stretch the lower fastening tape 70B outward in the width direction.

### (Bonded portions 91 and 92)

The right edge of the base portion 73 and the left edge of the main unit section 74 illustrated in Fig. 6 are bonded to each other in the dorsoventral direction by hot melt or the like. The bonded portion is referred to as a bonded portion 91. Similarly, the right edge of the main unit section 74 and the left edge of the engagement portion 76 are also bonded to each other in the dorsoventral direction by hot melt or the like. The portion bonded in the dorsoventral direction is referred to as a bonded portion 92. When the bonded portion 92 is cut along the slit portion 75, the bonded portion 92 is separated into the bonded portion 92A on the upper fastening tape 70A side and the bonded portion 92B on the lower fastening tape 70B side.

### (Separation along slit portion 75)

As illustrated in Figs. 9(a) and 9(b), when the disposable diaper 100 is worn by a wearer, first, a user cuts the fastening tape 70 along the slit portion 75, and separates the fastening tape 70 into the upper fastening tape 70A and the lower fastening tape 70B.

### (Stretch of lower fastening tape 70B)

Next, stretch of the fastening tape 70 will be described.

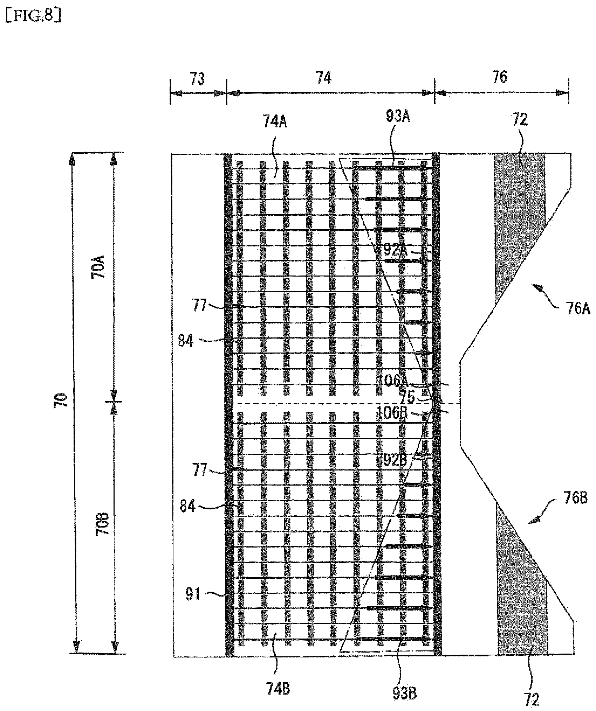

After separating the fastening tape 70 along the slit portion 75, as illustrated in Fig. 9(c), the user grasps the grip portion 103B of the engagement portion 76 and stretches the lower fastening tape 70B outward in the width direction. A force applied to the lower fastening tape 70B at this time will be described with reference to Fig. 8. This stretching force acts on the bonded portion 92B. At this time, the force acting on the bonded portion 92B is strong on a lower side of the bonded portion 92B and becomes weaker as it goes to an upper side. Regarding this action, the magnitude of the force acting on the bonded portion 92B is indicated by an arrow group 93B surrounded by a triangular one-dot chain line illustrated in Fig. 8. The arrow group 93B indicates that a longer arrow acts with a stronger force. In other words, a longer arrow indicates more stretch of the elastically stretchable member 77.

For example, when a user grasps the grip portion 103B and stretches the lower fastening tape 70B outward in the width direction, the lower side of the bonded portion 92B is pulled to the outside in the width direction with a relatively strong force. In this case, the elastically stretchable member 77 on the lower side of the main unit section 74B adjacent to the lower side of the bonded portion 92B stretches outward in the width direction.

Meanwhile, the upper side of the bonded portion 92B is pulled to the outside in the width direction with a relatively small force. In this case, the elastically stretchable member 77 on the upper side of the main unit section 74B adjacent to the upper side of the bonded portion 92B stretches outward in the width direction. However, a stretch width on the upper side of the main unit section 74B is smaller than a stretch width on the lower side of the main unit section 74B.

As described above, the stretch width of the main unit section 74B is large on the lower side of the main unit section 74B, and gradually decreases as it goes toward the upper side. Therefore, when a user grasps the grip portion 103B and tries to stretch the lower fastening tape 70B outward in the width direction, the lower fastening tape 70B is naturally curved so as to approach a dorsal side while stretching outward in the width direction.

### (Stretch of upper fastening tape 70A)

Subsequently, the user grasps the grip portion 103A of the engagement portion 76 and stretches the upper fastening tape 70A outward in the width direction. A force applied to the upper fastening tape 70A at this time will be described with reference to Fig. 8. This stretching force acts on the bonded portion 92A. At this time, the force acting on the bonded portion 92A is strong on an upper side of the bonded portion 92A and becomes weaker as it goes to a lower side. Regarding this action, the magnitude of the force acting on the bonded portion 92A is indicated by an arrow group 93A surrounded by a triangular one-dot chain line illustrated in Fig. 8. The arrow group 93A indicates that a longer arrow acts with a stronger force.

For example, when a user grasps the grip portion 103A and stretches the upper fastening tape 70A outward in the width direction, the upper side of the bonded portion 92A is pulled to the outside in the width direction with a relatively strong force. In this case, the elastically stretchable member 77 on the upper side of the main unit section 74A adjacent to the upper side of the bonded portion 92A stretches outward in the width direction.

Meanwhile, the lower side of the bonded portion 92A is pulled to the outside in the width direction with a relatively small force. In this case, the elastically stretchable member 77 on the lower side of the main unit section 74A adjacent to the lower side of the bonded portion 92A stretches outward in the width direction. However, a stretch width on the lower side of the main unit section 74A is smaller than a stretch width on the upper side of the main unit section 74A.

As described above, the stretch width of the main unit section 74A is large on the upper side of the main unit section 74A, and gradually decreases as it goes toward the lower side. Therefore, when a user grasps the grip portion 103A and tries to stretch the upper fastening tape 70A outward in the width direction, the upper fastening tape 70A is naturally curved so as to approach a ventral side while stretching outward in the width direction.

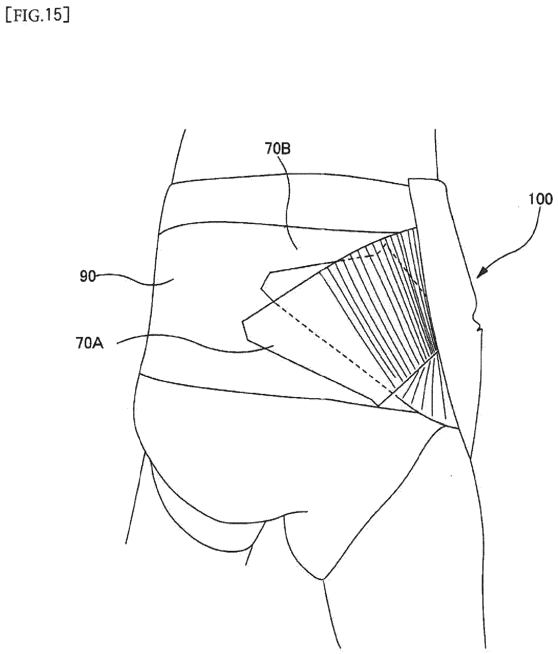

Through the above procedure, the user engages the fastening tape 70A and the fastening tape 70B with the target sheet 90 while the fastening tape 70A and the fastening tape 70B are crossed with each other as illustrated in Fig. 15. Specifically, the user extends the lower fastening tape 70B further upward as it is closer to the center of the target sheet 90 in the width direction. The user extends the upper fastening tape 70A further downward as it is closer to the center of the target sheet 90 in the width direction. Thereafter, the user engages the upper fastening tape 70A with the lower fastening tape 70B on the target sheet 90 so as to be crossed with each other in a substantially X shape.

### (Curvature)

The following advantages are obtained when the upper fastening tape 70A and the lower fastening tape 70B are curved. Specifically, when the upper fastening tape 70A and the lower fastening tape 70B are engaged with the target sheet 90 while the fastening tape 70A and the fastening tape 70B are crossed with each other in a substantially X shape, floating wrinkles are not formed on the upper fastening tape 70A or the lower fastening tape 70B.

When a first tape fastener and a second tape fastener disclosed in Patent Literature 1 that is related art are fixed to a target tape in a state where the first and second tape fasteners are inclined, floating wrinkles are formed at both ends of an inner end portion (31, 25) in a dorsoventral direction. These floating wrinkles are formed when the inner end portion (31, 25) does not expand or contract. Even if the inner end portion (31, 25) expands and contracts, the inner end portion (31, 25) does not become a fan shape described later, and both ends of the inner end portion (31, 25) in the dorsoventral direction are warped to form floating wrinkles.

Meanwhile, in the lower fastening tape 70B of the present embodiment, the grip portion 103B is located closer to the engagement portion lower end edge 102B. When a user grasps the grip portion 103B and tries to stretch the lower fastening tape 70B outward in the width direction, a force to stretch the lower fastening tape 70B is relatively strongly transmitted to the lower side of the bonded portion 92B because a linear distance between the grip portion 103B and the lower side of the bonded portion 92B is relatively short. Meanwhile, a force to stretch the lower fastening tape 70B is relatively weakly transmitted to the upper side of the bonded portion 92B because a linear distance between the grip portion 103B and the upper side of the bonded portion 92B is relatively long. The lower side of the main unit section 74B stretches more than the upper side (slit portion 75 side) of the main unit section 74B, and the main unit section 74B becomes a fan shape having a substantially arc-shaped lower side. Since the main unit section 74B becomes a fan shape, the stretching force is appropriately dispersed, and no floating wrinkles are formed on the lower fastening tape 70B.

The upper fastening tape 70A and the lower fastening tape 70B of the present embodiment are substantially line-symmetrical with the slit portion 75 as an axis. Therefore, in the upper fastening tape 70A, the grip portion 103A is located closer to the engagement portion upper end edge 102A. When a user grasps the grip portion 103A and tries to stretch the upper fastening tape 70A outward in the width direction, a force to stretch the upper fastening tape 70A is relatively strongly transmitted to the upper side of the bonded portion 92A because a linear distance between the grip portion 103A and the upper side of the bonded portion 92A is relatively short. Meanwhile, a force to stretch the upper fastening tape 70A is relatively weakly transmitted to the lower side of the bonded portion 92A because a linear distance between the grip portion 103A and the lower side of the bonded portion 92A is relatively long. The upper side of the main unit section 74A stretches more than the lower side (slit portion 75 side) of the main unit section 74A, and the main unit section 74A becomes a fan shape having a substantially arc-shaped upper side. Since the main unit section 74A becomes a fan shape, the stretching force is appropriately dispersed, and no floating wrinkles are formed on the upper fastening tape 70A.

As described above, if floating wrinkles are not formed, an inner surface of the disposable diaper 100 fits to a wearer more securely, and excrement does not easily leak to the outside of the disposable diaper 100. In addition, even if the posture of the wearer changes, the main unit section 74A freely expands and contracts in the width direction, and therefore the fit of the disposable diaper 100 to the wearer is maintained.

### <Fastening tape 70 of second embodiment>

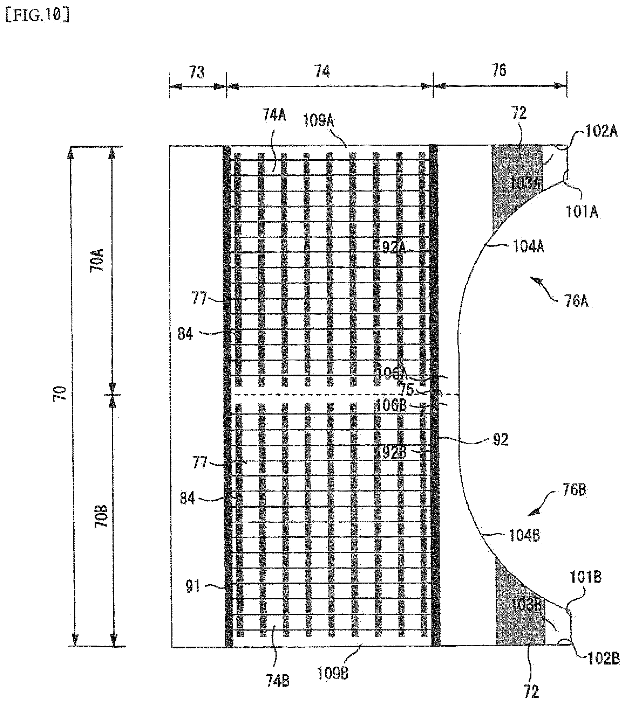

As illustrated in Fig. 10, a fastening tape of a second embodiment has a form in which the engagement portion inclined edge (104A, 104B) of the fastening tape of the first embodiment is changed into a curved line. The curved line preferably protrudes toward the central side in the width direction. The shape of the curved line is not particularly limited, and can be, for example, a substantially circular arc, a substantially elliptical arc, or a substantially multiple curved line.

### (Separation at slit portion 75)

When a disposable diaper 100 including a fastening tape 70 of the second embodiment is worn by a wearer, first, a user cuts the fastening tape 70 at a slit portion 75, and separates the fastening tape 70 into an upper fastening tape 70A and a lower fastening tape 70B.

### (Stretch of fastening tape 70)

After separating the fastening tape 70 at the slit portion 75, the user grasps a grip portion 103B of an engagement portion 76 and stretches the lower fastening tape 70B outward in the width direction. At this time, since an engagement portion inclined edge 104B is a substantially curved line from the grip portion 103B to a narrowing portion 106B, this stretching force acts on an entire bonded portion 92B. As described above for the fastening tape of the first embodiment, the force acting on the bonded portion 92B is relatively strong on a lower side of the bonded portion 92B, and gradually decreases as it goes to an upper side. The lower side of a main unit section 74B stretches more than the upper side (slit portion 75 side) of the main unit section 74B, and the main unit section 74B becomes a fan shape having an arc-shaped lower side. Since the main unit section 74B becomes a fan shape, no floating wrinkles are formed on the lower fastening tape 70B.

After engaging the lower fastening tape 70B with a target sheet 90, the user grasps a grip portion 103A of the engagement portion 76 and stretches the upper fastening tape 70A outward in the width direction. At this time, since an engagement portion lower end edge 104A is a substantially curved line from the grip portion 103A to a narrowing portion 106A, this stretching force acts on an entire bonded portion 92A. The force acting on the bonded portion 92A is relatively strong on the upper side of the bonded portion 92A, and gradually becomes weaker as it goes to a lower side. The upper side of the main unit section 74A stretches more than the lower side (slit portion 75 side) of the main unit section 74A, and the main unit section 74A becomes a fan shape having a substantially arc-shaped upper side. Since the main unit section 74A becomes a fan shape, the stretching force is appropriately dispersed, and no floating wrinkles are formed on the upper fastening tape 70A.

In the second embodiment, since the engagement portion inclined edge (104A, 104B) of the fastening tape 70 is a substantially curved line, first, the area of the engagement portion 76 of the second embodiment is smaller than the area of the engagement portion 76 of the fastening tape 70 of the first embodiment. Second, the engagement portion 76A and the engagement portion 76B of the second embodiment are relatively unevenly distributed on the engagement portion upper end edge 102A side and the engagement portion lower end edge 102B side, respectively. As described above, since the engagement portion (76A, 76B) is unevenly distributed, a user is guided to naturally grasp the engagement portion upper end edge 102A side and the engagement portion lower end edge 102B side.

### <Fastening tape 70 of third embodiment>

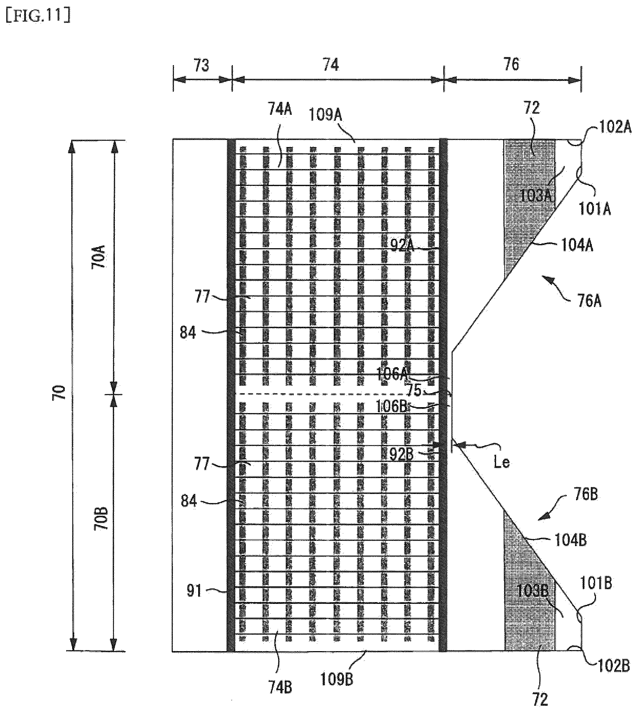

As illustrated in Fig. 11, a fastening tape 70 of a third embodiment has a form in which the thickness Le of the narrowing portion of the semi-hourglass shape in the fastening tape 70 of the first embodiment is reduced.

### (Separation at slit portion 75)

A user cuts the fastening tape 70 of the third embodiment at a slit portion 75, and separates the fastening tape 70 into an upper fastening tape 70A and a lower fastening tape 70B.

### (Stretch of fastening tape 70)

After separating the fastening tape 70 at the slit portion 75, the user grasps a grip portion 103B of an engagement portion 76 and stretches the lower fastening tape 70B outward in the width direction. This stretching force acts on an entire bonded portion 92B. At this time, the force acting on the bonded portion 92B is relatively strong on a lower side of the bonded portion 92B and becomes weaker as it goes to an upper side. Here, since the thickness Le of the narrowing portion of the semi-hourglass shape in the fastening tape 70 of the third embodiment is relatively thin, the force acting on the upper side of the bonded portion 92B of the third embodiment is weaker than the force acting on the upper side of the bonded portion 92B of the first embodiment. As a result, a stretch width on an upper side of a main unit section 74B of the third embodiment is smaller than a stretch width on an upper side of the main unit section 74B of the first embodiment.

Therefore, when a user grasps the grip portion 103B and tries to stretch the lower fastening tape 70B outward in the width direction, the curvature of the lower fastening tape 70B of the third embodiment becomes steeper than the curvature of the lower fastening tape 70B of the first embodiment. That is, the lower fastening tape 70B is curved so as to further approach a dorsal side.

After engaging the lower fastening tape 70B with a target sheet 90, the user grasps a grip portion 103A of the engagement portion 76 and stretches the upper fastening tape 70A outward in the width direction. At this time, this stretching force acts on an entire bonded portion 92A. The force acting on the bonded portion 92A is relatively strong on the upper side of the bonded portion 92A, and gradually becomes weaker as it goes to a lower side. Here, since the thickness Le of the of the semi-hourglass shape narrowing portion in the fastening tape 70 of the third embodiment is relatively thin, the force acting on the lower side of the bonded portion 92A is weaker than the force acting on the lower side of the bonded portion 92A of the first embodiment. As a result, a stretch width on a lower side of a main unit section 74B of the third embodiment is smaller than a stretch width on a lower side of the main unit section 74B of the first embodiment.

Therefore, when a user grasps the grip portion 103A and tries to stretch the upper fastening tape 70A outward in the width direction, the curvature of the upper fastening tape 70A of the third embodiment becomes steeper than the curvature of the lower fastening tape 70A of the third embodiment. That is, the upper fastening tape 70A is curved so as to further approach a ventral side. By such a form, the user is guided to perform cross-fastening for the upper fastening tape 70A to the target sheet 90.

### <Fastening tape 70 of fourth embodiment>

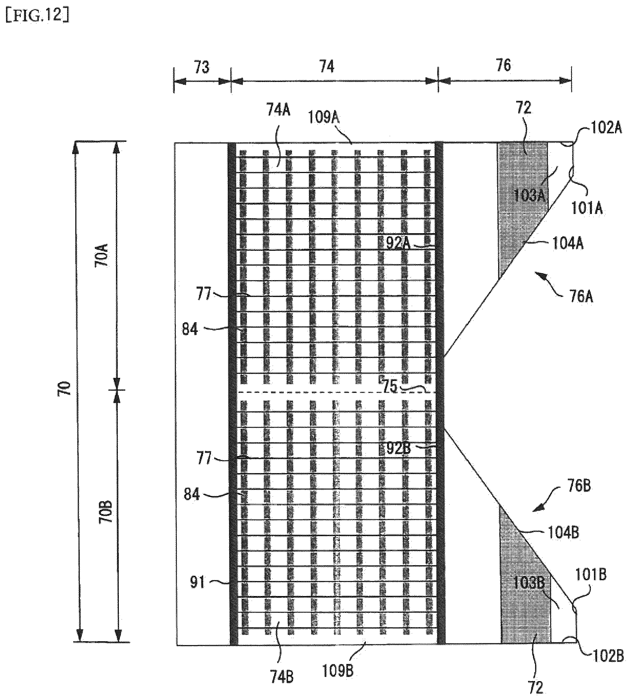

As illustrated in Fig. 12, a fastening tape 70 of a fourth embodiment has a form in which the thickness Le of the narrowing portion of the semi-hourglass shape in the fastening tape 70 of the first embodiment is zero. That is, the fastening tape 70 of the fourth embodiment has a form in which an end edge of a narrowing portion (106A, 106B) overlaps an end edge of a main body 74 in the width direction. Specifically, the fastening tape 70 of the fourth embodiment has a form in which the length Lg of an engagement portion in the width direction is equal to the length Lf from an engagement portion outer edge 101 to a narrowing portion end edge 107. Here, the length Lb from an engagement portion upper end edge 102A (or engagement portion lower end edge 102B) to the narrowing portion of the semi-hourglass shape is preferably equal to or less than the length Lc of an upper fastening tape 70A (or lower fastening tape 70B).

Note that the fastening tape 70 of the fourth embodiment includes a form in which the width of the narrowing portion (106A, 106B) in the dorsoventral direction is 0, and a form in which the length Lc of the upper fastening tape 70A (or lower fastening tape 70B) in the dorsoventral direction is equal to the length Lb from the engagement portion upper end edge 102A (or engagement portion lower end edge 102B) to the narrowing portion of the semi-hourglass shape.

### (Stretch of fastening tape 70)

A user cuts the fastening tape 70 of the fourth embodiment at a slit portion 75, and separates the fastening tape 70 into an upper fastening tape 70A and a lower fastening tape 70B.

After separating the fastening tape 70 at the slit portion 75, the user grasps a grip portion 103B of an engagement portion 76 and stretches the lower fastening tape 70B outward in the width direction. This stretching force acts on the bonded portion 92B. At this time, the force acting on the bonded portion 92B is relatively strong on a lower side of the bonded portion 92B and becomes weaker as it goes to an upper side. However, in the fourth embodiment, since the thickness Le of the narrowing portion 106B is 0, the force acting on an upper side of the bonded portion 92B adjacent to the narrowing portion 106B having a thickness of 0 is still weaker than the force acting on the upper side of the bonded portion 92B of the third embodiment.

Therefore, when a user grasps the grip portion 103B and tries to stretch the lower fastening tape 70B outward in the width direction, the curvature of the lower fastening tape 70B of the fourth embodiment becomes still steeper than the curvature of the lower fastening tape 70B of the third embodiment. That is, the lower fastening tape 70B is further curved so as to approach a dorsal side.

After engaging the upper fastening tape 70A with a target sheet 90, the user grasps a grip portion 103A of the engagement portion 76 and stretches the upper fastening tape 70A outward in the width direction. This stretching force acts on the bonded portion 92A. At this time, the force acting on the bonded portion 92A is relatively strong on the upper side of the bonded portion 92A, and gradually becomes weaker as it goes to a lower side. However, since the thickness Le of the narrowing portion is 0, the force acting on a lower side of the bonded portion 92A adjacent to the narrowing portion 106A having a thickness of 0 is still weaker than the force acting on the lower side of the bonded portion 92A of the third embodiment.

Therefore, when a user grasps the grip portion 103A and tries to stretch the upper fastening tape 70A outward in the width direction, the curvature of the upper fastening tape 70A of the fourth embodiment becomes still steeper than the curvature of the upper fastening tape 70A of the third embodiment. That is, the upper fastening tape 70A is further curved so as to approach a ventral side. By such a form, the user is guided to perform cross-fastening for the upper fastening tape 70A to the target sheet 90.

### <Fastening tape 70 of fifth embodiment>

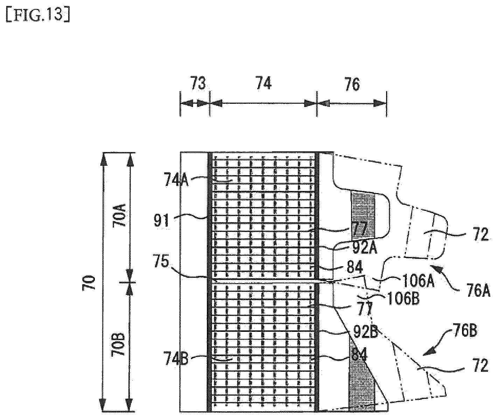

A fastening tape 70 of a fifth embodiment has a form in which only a lower engagement portion 76B has the same shape as the lower engagement portion 76B of any one of the first to fourth embodiments. The thickness Le of a narrowing portion of the lower engagement portion 76B is 0 or more and less than the length Lg thereof in the width direction. Meanwhile, the form of an upper engagement portion 76A of the fifth embodiment is not limited to the form of the upper engagement portion 76A of any one of the first to fourth embodiments. That is, the form of the upper engagement portion 76A is not limited to a form in which curvature of an upper fastening tape 70A is intended, but may be a known form as long as the upper engagement portion 76A protrudes outward from a main unit section 74A in the width direction. For example, as illustrated in Fig. 13, the upper engagement portion 76A can have a form in which the central portion of the upper engagement portion 76A in the dorsoventral direction protrudes outward in the width direction.

An advantage due to a fact that the lower fastening tape 70B stretches while being curved has been described in the first to fourth embodiments. Similarly, in the lower fastening tape 70B of the fifth embodiment, the lower fastening tape 70B stretches while being curved. In the fifth embodiment, when a user grasps a grip portion 103B and tries to stretch the lower fastening tape 70B outward in the width direction, the lower fastening tape 70B is naturally curved so as to approach a dorsal side while stretching outward in the width direction. Then, a user engages the lower fastening tape 70B with a top surface of the target sheet 90. When the user engages the lower fastening tape 70B in this manner, an elastically stretchable member 77 on a lower side of the main unit section 74B of the lower fastening tape 70B relatively largely stretches. The stretch of the elastically stretchable member 77 gradually decreases as it goes toward an upper side of the main unit section 74B. Therefore, a contraction force of the elastically stretchable member 77 acting on an upper side of the lower fastening tape 70B (near the slit portion 75) is relatively weak.

Therefore, the user engages the lower fastening tape 70B with a top surface of the target sheet 90, and then the user preferably covers the lower fastening tape 70B with the upper fastening tape 70A from above while the upper fastening tape 70A is stretched outward in the width direction to engage the lower fastening tape 70B with the upper fastening tape 70A. That is, the lower fastening tape 70B and the upper fastening tape 70A are engaged with each other such that the upper fastening tape 70A is crossed with the lower fastening tape 70B. In this case, a portion of the elastically stretchable member 77 where the contraction force is weak (near the slit portion 75) is pressed by the upper fastening tape 70A. As described above, by covering the vicinity of the slit portion 75 of the lower fastening tape 70B with the upper fastening tape 70A, the weak contraction force of the elastically stretchable member 77 can be reinforced. With this reinforcement, floating wrinkles of the lower fastening tape 70B and curling thereof can be reliably prevented. As a result, the disposable diaper securely fits the wearer.

### (Manufacturing method)

A cutting step in a process for manufacturing the fastening tape 70 will be described below.

As illustrated in Fig. 14, in the fastening tape 70, an engagement portion, a main unit section, and a base portion extend in a manufacturing direction. The engagement portion 76 is disposed at the center in the manufacturing width direction WD. The base portions 73 are disposed at both end edges in the manufacturing width direction WD. The main unit section is disposed so as to be sandwiched between the engagement portion 76 and each of the base portions 73 and to be adjacent thereto. At the center of the engagement portion 76 in the manufacturing width direction WD, the engagement member 72 extends in the manufacturing direction LD. The width of the engagement member 72 in the manufacturing width direction WD may be the same as the width of the engagement portion 76, or may be shorter than the width of the engagement portion 76. Here, for the sake of explanation, the fastening tape 70 located on the left side of a cutting portion 111 in the manufacturing width direction WD in Fig. 14 is referred to as a left fastening tape 70L, and the fastening tape 70 located on the right side thereof is referred to as a right fastening tape 70R.

The cutting portion 111 is an uneven polygonal line disposed at the center of the engagement portion 76 in the manufacturing width direction WD and extending in the manufacturing direction LD. The shape of the uneven polygonal line is as follows. First, two broken lines (111α and 111β) extending in parallel at intervals are imagined. The two broken lines (111α and 111β) extend alternately. That is, the two broken lines (111α and 111β) extend in a staggered manner. Each end point of the broken line 111α is connected to an end point of the broken line 111β, which is the closest from the end point of the broken line 111α, with a line 111γ. When the broken line 111α and the broken line 111β are connected to each other in this manner, a single solid line 111 is formed. Here, if a solid line portion of each of the broken lines (111α and 111β) is short, the line 111γ is oblique to the manufacturing direction LD.

Examples of the solid line 111 include the cutting portion 111 in Fig. 14. The cutting portion 111 is formed so as to have a portion including, from an upper side to a lower side in the manufacturing direction LD, a cutting portion 111α, the following cutting portion 111γ, the following cutting portion 111β, the following cutting portion 111γ, and the following cutting portion 111α, as one cycle. The fastening tape 70 is formed so as to have the one cycle as one unit.

The illustrated cutting portion 110L extends from a left end portion of the left fastening tape 70L in the manufacturing width direction WD to a cutting portion 111β (narrowing portion end edge 107) in the manufacturing width direction WD. Following the cutting portion 110L, a slit portion 75R extends in the right fastening tape 70R in the manufacturing width direction WD. The slit portion 75R extends from the cutting portion 111β (narrowing portion end edge 107) to a bonded portion 91R.

The cutting portion 110L and the slit portion 75R following the cutting portion 110L are disposed for each unit length of the left fastening tape 70L in the manufacturing direction LD. Here, the length of one unit of the left fastening tape 70L in the manufacturing direction LD refers to the total of the length of the fastening tape 70A in the manufacturing direction LD and the length of the fastening tape 70B in the manufacturing direction LD.

At a boundary between an upper fastening tape 70A1 and a lower fastening tape 70B1 on the left side in the manufacturing direction LD, a slit portion 75L extends from a bonded portion 91L to a cutting portion 111β (narrowing portion end edge 107) in the manufacturing width direction WD. Following the slit portion 75L, a cutting portion 110R extends from the cutting portion 111β (narrowing portion end edge 107) to a right end portion of the right fastening tape 70R in the manufacturing width direction WD. The slit portion 75L and the cutting portion 110R following the slit portion 75L are disposed for each unit length of the left fastening tape 70L in the manufacturing direction LD.

When the cutting portion 110L and the slit portion 75R are disposed in this manner, as it goes from the upper side to the lower side in the manufacturing direction LD, at a boundary between a lower fastening tape 70B0 and the upper fastening tape 70A1, "the cutting portion 110L and the slit portion 75R following the cutting portion 110L" are disposed in the manufacturing width direction WD. When it goes further downward, at a boundary between the upper fastening tape 70A1 and the lower fastening tape 70B1, "the slit portion 75L and the cutting portion 110R following the slit portion 75L" are disposed in the manufacturing width direction WD. When it goes further downward, at a boundary between the lower fastening tape 70B1 and an upper fastening tape 70A2, "the cutting portion 110L and the slit portion 75R following the cutting portion 110L" are disposed in the manufacturing width direction WD. Thereafter, the "the cutting portion 110L and the slit portion 75R following the cutting portion 110L" and "the slit portion 75L and the cutting portion 110R following the slit portion 75L" are alternately disposed at an interval of the fastening tape 70A (or fastening tape 70B) in the manufacturing direction LD. Note that in the cutting step, the fastening tape 70 is cut into units at the cutting portions (110L, 110R, and 111).

Therefore, from the continuous fastening tape 70, both the left fastening tape 70L and the right fastening tape 70R are manufactured at one time. In the manufacturing process, no portion to be discarded by cutting the fastening tape 70 is generated, and there is no loss of materials of the fastening tape, which is economical.

### Industrial Applicability

The present invention is applicable to a tape-type disposable diaper.

### Reference Signs List

- 1: Top sheet
- 2: Back sheet
- 3: Absorber
- 20: Outer sheet
- 70: Fastening tape
- 70A: Upper fastening tape
- 70B: Lower fastening tape
- 70L: Left fastening tape
- 70R: Right fastening tape
- 71: Substrate sheet
- 71A: Inner substrate sheet
- 71B: Outer substrate sheet
- 72: Engagement member
- 73: Base portion
- 74: Main unit section
- 74A: Main unit section
- 74B: Main unit section
- 75: Slit portion
- 75L: Slit portion
- 75R: Slit portion
- 76: Engagement portion
- 76A: Upper engagement portion
- 76B: Lower engagement portion
- 77: Elastically stretchable member
- 90: Target sheet
- 91: Bonded portion
- 92: Bonded portion
- 92A: Bonded portion
- 92B: Bonded portion
- 100: Disposable diaper
- 101A: Engagement portion outer edge
- 101B: Engagement portion outer edge
- 102A: Engagement portion upper end edge
- 102B: Engagement portion lower end edge
- 103A: Grip portion
- 103B: Grip portion
- 104A: Engagement portion inclined edge
- 104B: Engagement portion inclined edge
- 106A: Narrowing portion
- 106B: Narrowing portion
- 107: Narrowing portion end edge
- 109A: Upper end edge of main unit section
- 109B: Lower end edge of main unit section
- 110: Cutting portion
- 110L: Cutting portion
- 110R: Cutting portion
- 111: Cutting portion
- La: Length of engagement portion outer edge 101A (or engagement portion outer edge 101B)
- Lb: Length from engagement portion upper end edge 102A (or engagement portion lower end edge 102B) to narrowing portion of semi-hourglass shape
- Lc: Length of upper fastening tape 70A (or lower fastening tape 70B) in dorsoventral direction
- Le: Thickness of the narrowing portion of the semi-hourglass shape
- Lf: Length from engagement portion outer edge 101 to narrowing portion end edge 107
- Lg: Length of engagement portion in width direction
- LD: Manufacturing direction
- WD: Manufacturing width direction

## Claims

1. A disposable diaper (100) comprising:
a liquid pervious top sheet (1);
a liquid impervious back sheet (2);
an absorber (3) disposed between the top sheet (1) and the back sheet (2); and
an outer sheet (20) disposed from a dorsal side to a ventral side on a back surface of the back sheet (2),
wherein a fastening tape (70) extending in a direction orthogonal to a dorsoventral direction is disposed in a dorsal section of the outer sheet (20),
the fastening tape (70) includes: a base portion (73) fixed to the outer sheet (20); a main unit section (74) located on a distal end of the base portion (73); and an engagement portion (76) located on a distal end of the main unit section (74),
a dorsal end edge of the main unit section (74) and a dorsal end edge of the engagement portion (76) are continuous and extend adjacent each other on the same straight line in a direction orthogonal to a dorsoventral direction,
a ventral end edge of the main unit section (74) and a ventral end edge of the engagement portion (76) are continuous and extend adjacent each other on the same straight line in a direction orthogonal to a dorsoventral direction,
the fastening tape (70) has a slit portion (75) formed in an extending direction,
the slit portion (75) separating the fastening tape (70) into the upper fastening tape (70A) and the lower fastening tape (70B),
the main unit section (74) has elastically stretchable members (77) arranged in the extending direction at intervals in the dorsoventral direction, and
a ventral portion and a dorsal portion in the engagement portion (76) extend more distally than a central portion thereof,
distal portions of the dorsal portion and the ventral portion in the engagement portion (76) are each a grip portion (103A, 103B), and
an extension length of the engagement portion (76) becomes shorter as it goes from the grip portion (103A, 103B) to the central portion.

2. The disposable diaper according to claim 1,
wherein a leading edge of the engagement portion (76) overlaps a leading edge of the main unit section (74) in the central portion.

## Patentansprüche

1. Wegwerfwindel, (100) umfassend:
eine flüssigkeitsdurchlässige Decklage (1);
eine flüssigkeitsundurchlässige hintere Lage (2);
einen Absorber (3), der zwischen der Decklage (1) und der hinteren Lage (2) vorgesehen ist; und
eine äußere Lage (20), die von einer Rückenseite zu einer Bauchseite auf einer hinteren Oberfläche der hinteren Lage (2) vorgesehen ist,
wobei ein Befestigungsband (70), das sich in einer Richtung orthogonal zu einer Richtung vom Rücken zum Bauch erstreckt, in einem Rückenabschnitt der äußeren Lage (20) vorgesehen ist,
das Befestigungsband (70) umfasst: einen Basisabschnitt (73), der an der äußeren Lage (20) befestigt ist; einen Haupteinheitsabschnitt (74), der sich an einem distalen Ende des Basisabschnitts (73) befindet, und einen Eingriffsabschnitt (76), der sich an einem distalen Ende des Haupteinheitsabschnitts (74) befindet,
eine rückenseitige Endkante des Haupteinheitsabschnitts (74) und eine rückenseitige Endkante des Eingriffsabschnitts (76) durchgehend sind und sich nebeneinander auf derselben geraden Linie in einer Richtung orthogonal zu einer Richtung vom Rücken zum Bauch erstrecken,
eine bauchseitige Endkante des Haupteinheitsabschnitts (74) und eine bauchseitige Endkante des Eingriffsabschnitts (76) durchgehend sind und sich nebeneinander auf derselben geraden Linie in einer Richtung orthogonal zu einer Richtung vom Rücken zum Bauch erstrecken,
das Befestigungsband (70) einen Schlitzabschnitt (75) aufweist, der in einer Erstreckungsrichtung ausgebildet ist,
der Schlitzabschnitt (75) das Befestigungsband (70) in das obere Befestigungsband (70A) und das untere Befestigungsband (70B) trennt,
der Haupteinheitsabschnitt (74) elastisch dehnbare Elemente (77) aufweist, die in der Erstreckungsrichtung in Abständen in einer Richtung vom Rücken zum Bauch angeordnet sind, und
ein Bauchabschnitt und ein Rückenabschnitt im Eingriffsabschnitt (76) sich weiter distal erstrecken als ein mittlerer Abschnitt davon,
distale Abschnitte des Rückenabschnitts und des Bauchabschnitts im Eingriffsabschnitt (76) jeweils ein Griffabschnitt (103A, 103B) sind, und
eine Erstreckungslänge des Eingriffsabschnitts (76) im Verlauf von dem Griffabschnitt (103A, 103B) zum mittleren Abschnitt kürzer wird.

2. Wegwerfwindel nach Anspruch 1,
wobei eine Vorderkante des Eingriffsabschnitts (76) im mittleren Abschnitt mit einer Vorderkante des Haupteinheitsabschnitts (74) überlappt.

## Revendications

1. Couche jetable (100) comprenant :
une feuille de dessus (1) perméable au liquide ;
une feuille arrière imperméable aux liquides (2) ;
un absorbeur (3) disposé entre la feuille de dessus (1) et la feuille arrière (2) ; et
une feuille externe (20) disposée d'un côté dorsal à un côté ventral sur une surface arrière de la feuille arrière (2),
dans laquelle une bande de fixation (70) s'étendant dans une direction orthogonale à une direction dorsoventrale est disposée dans une section dorsale de la feuille externe (20),
la bande de fixation (70) inclut : une portion de base (73) arrimée à la feuille externe (20) ; une section d'unité principale (74) située sur une extrémité distale de la portion de base (73) ; et une portion d'engagement (76) située sur une extrémité distale de la section d'unité principale (74),
un bord d'extrémité dorsal de la section d'unité principale (74) et un bord d'extrémité dorsal de la portion d'engagement (76) sont continus et s'étendent adjacents l'un à l'autre sur la même droite dans une direction orthogonale à une direction dorsoventrale,
un bord d'extrémité ventral de la section d'unité principale (74) et un bord d'extrémité ventral de la portion d'engagement (76) sont continus et s'étendent adjacents l'un à l'autre sur la même droite dans une direction orthogonale à une direction dorsoventrale,
la bande de fixation (70) présente une portion fendue (75) formée dans une direction d'extension,
la portion fendue (75) séparant la bande de fixation (70) en la bande de fixation supérieure (70A) et la bande de fixation inférieure (70B),
la section d'unité principale (74) comporte des organes (77) élastiquement extensibles agencés dans la direction d'extension à intervalles dans la direction dorsoventrale, et
une portion ventrale et une portion dorsale dans la portion d'engagement (76) s'étendent plus distalement qu'une portion centrale de celle-ci,
les portions distales de la portion dorsale et de la portion ventrale dans la portion d'engagement (76) sont chacune une portion de préhension (103A, 103B), et
une longueur d'extension de la portion d'engagement (76) devient plus courte lorsqu'elle va de la partie de préhension (103A, 103B) à la portion centrale.

2. Couche jetable selon la revendication 1,
dans laquelle un bord d'attaque de la portion d'engagement (76) chevauche un bord d'attaque de la section d'unité principale (74) dans la portion centrale.
